# FASCICULE DE BREVET EUROPEEN

(11) **EP 0 600 775 B1**
(45) Date de publication et mention de la délivrance du brevet: **07.05.1997**
(21) Numéro de dépôt: 93402877.0
(22) Date de dépôt: 29.11.1993
(51) Int. Cl.: A61K 9/28, A61K 7/00, A23P 1/08

(54) **Principes actifs pulvérulents stabilisés, compositions les contenant, leur procédé d'obtention et leurs applications**
Pulverförmige, stabilisierte Wirkstoffe, diese enthaltende Zusammensetzungen Verfahren zu ihrer Herstellung und ihre Verwendung
Powdery, stabilized active agents, composition containing them, method for producing them and their use

(30) Priorité: 30.11.1992 FR 9214365
(43) Date de publication de la demande: 08.06.1994
(73) Titulaire: LABORATOIRES VIRBAC, 06516 Carros (FR)
(72) Inventeur: Derrieu, Guy, F-06800 Cagnes Sur Mer (FR); Raynier, Bernard, F-06200 Nice (FR)
(74) Mandataire: Orès, Bernard

(56) Documents cités:
- FR-A- 2 447 720
- FR-A- 2 548 675
- FR-A- 2 657 255
- GB-A- 2 133 983
- US-A- 4 687 660

## Description

La présente invention est relative à des principes actifs pulvérulents stabilisés, aux compositions les contenant ainsi qu'à leur procédé d'obtention ; de tels principes actifs stabilisés permettent d'obtenir des compositions pharmaceutiques, diététiques, alimentaires ou cosmétiques, sèches et stables, dans lesquelles les propriétés desdits principes actifs ne sont ni modifiées, ni altérées.

On entend, au sens de la présente invention, par principe actif, toute substance chimique, susceptible de se transformer, se dégrader ou s'altérer, sous l'influence d'une quelconque manipulation physique, mécanique ou thermique, en particulier sous pression et en présence d'eau.

En effet de telles transformations ne permettent pas de maintenir l'intégrité des principes actifs, lors de la production et de la conservation dans le temps de produits manufacturés tels que des mélanges de poudres et des compositions granulées ou compactées.

La présentation de nombreux principes actifs, sous forme de granulés ou de microcapsules, sphériques ou non, enrobés, pour, entre autres avantages, en faciliter l'écoulement, le transfert, éviter l'adhésion interparticulaire ou obtenir des effets retards, est connu de l'Homme du métier.

Les microcapsules notamment sont généralement préparées selon le procédé de coacervation simple, dans lequel un polymère se sépare d'une solution du polymère dans un solvant, soit par évaporation dudit solvant, soit par l'action d'un agent de précipitation (sel ou non-solvant du dit polymère). Une autre technique met en jeu une microencapsulation basée sur une polymérisation par condensation interfaciale *in situ.*

Toutefois, de tels enrobages ne sont pas adaptés à la stabilisation, sans modification des caractéristiques physiques, en particulier, la solubilité, la lipophilie et le passage des différentes barrières physiologiques, des principes actifs traités.

La présente invention s'est en conséquence donné pour but de pourvoir à des principes actifs stabilisés ainsi qu'aux compositions pulvérulentes contenant ces principes stabilisés ; en effet, la stabilisation permet d'éviter toute altération de ces principes actifs pendant les opérations de formulation, telles que la granulation ou le compactage, pour leur mise en forme finale (pharmaceutique, diététique, alimentaire ou cosmétique) et garantit l'intégrité des principes actifs, tout au long de la durée de vie des produits manufacturés obtenus, sans en modifier leur activité et leurs propriétés physiques.

La présente invention a pour objet des principes actifs pulvérulents stabilisés par enrobage avec une composition d'enrobage du type comprenant au moins un agent filmogène, caractérisés :
(i) en ce que ladite composition d'enrobage comprend :
   - au moins un agent filmogène, dans des proportions comprises entre 2 et 25 % (en poids) de la masse finale, sélectionné parmi les polyvinylpyrrolidones (povidone), les alcools polyvinyliques, le copolymère vinylpyrrolidone - acétate de vinyle, le copolymère vinylpyrrolidone - alcool polyvinylique, les dérivés cellulosiques tels que cellulose acétate, cellulose acétate phtalate, cellulose butyrate, éthylcellulose, méthylcellulose, les polymères et copolymères acryliques et méthacryliques et les cires végétales, (Carnauba, Candellila...), animales (abeille, huile de ricin hydrogénée...) ou synthétiques (polymères éthyléniques, polyol ether-ester tel que Carbowax®...), et
   - au moins un agent porogène, dans des proportions comprises entre 0 et 5 %, de préférence entre 0,5 et 5 %, en poids de la masse finale, sélectionné parmi le lactose microcristallin, les polyéthylènes glycols de bas poids moléculaire, le carbonate de calcium, le phosphate de calcium, le saccharose, le chlorure de sodium et le chlorure de potassium,
(ii) et en ce que le produit actif pulvérulent stabilisé sous la forme de microparticules, présente une granulométrie comprise entre 50 et 1 000 µm, de préférence entre 200 et 500 µm.

On entend par masse finale, le poids du produit fini (principe actif enrobé).

Lesdits principes actifs sont, de préférence, enrobés par pulvérisation de ladite composition d'enrobage en solution, en suspension ou en émulsion dans un véhicule ou un mélange de véhicules inertes vis-à-vis du principe actif, sur ledit principe actif sous forme pulvérulente.

Ladite composition d'enrobage comprenant un agent filmogène, présent dans des proportions comprises entre 2 et 25 % en poids de la masse finale, de préférence entre 5 et 15 %, et éventuellement un agent porogène présent dans des proportions comprises entre 0,5 et 5 % en poids de la masse finale, est apte à former une pellicule uniforme à la surface des principes actifs pulvérulents.

Ces principes actifs stabilisés, sous forme pulvérulente, c'est-à-dire enrobés dans une composition d'enrobage comprenant au moins un agent filmogène et, éventuellement, au moins un agent porogène, forment des microparticules dans lesquelles les principes actifs sont efficacement protégés des traitements physiques ultérieurs (notamment chaleur), mis en oeuvre pour leur mise en forme finale, (notamment granulation et compactage), tout en ne modifiant pas leurs propriétés physiques (solubilité, lipophilie).

En outre, l'association agent filmogène et agent porogène permet d'obtenir une composition d'enrobage dont la structure polymérique (agent filmogène), est modifiée par la présence de l'agent porogène ; une telle composition d'enrobage présente en conséquence les caractéristiques particulières suivantes :
. pendant l'étape de préparation des compositions granulées ou compactées comprenant lesdits principes actifs stabilisés sous forme de microparticules : résistance de ladite composition d'enrobage, lors de l'opération de granulation (même en présence de vapeur d'eau), car l'agent porogène, dont la structure est essentiellement cristalline, n'est pas modifié pendant cette opération (pas de dissolution de l'agent porogène) ; en conséquence, il n'y a pas destruction de l'enrobage et le principe actif est bien protégé ; en effet, de tels principes actifs stabilisés ne sont pas dégradés lors de l'opération de granulation par extrusion notamment, dans laquelle l'action conjuguée de solvants et de températures élevées, préférentiellement en présence d'eau ou de vapeur d'eau, avec ou sans générateur de pression, peut intervenir ; alors que
. lors de l'administration orale desdites compositions granulées ou compactées comprenant lesdits principes actifs enrobés, il y a destruction rapide de ladite composition d'enrobage, du fait de la présence dudit agent porogène, constituant des points d'accès préférentiels aux fluides biologiques.

Les principes actifs enrobés avec une composition d'enrobage conforme à l'invention, outre le fait qu'ils sont stabilisés, conservent, de manière surprenante, la propriété de se solubiliser normalement, c'est-à-dire à la même vitesse que le principe actif non enrobé (absence d'effet retard).

Selon un mode de réalisation avantageux de l'invention, préalablement à leur enrobage par ladite composition d'enrobage, lesdits principes actifs peuvent être associés à un agent séquestrant.

Les agents séquestrants sont notamment choisis parmi les carbohydrates ou polysaccharides tels que cellulose, dextrines, cyclodextrines, amidon, dextranes...

L'agent filmogène et, éventuellement, l'agent porogène et l'agent séquestrant sont choisis selon la nature du principe actif à stabiliser, sans pour autant, en aucun cas, modifier les caractéristiques physiques précitées du principe actif.

Les principes actifs sont préférentiellement choisis dans les familles suivantes :
- vitamines, telles que thiamine (sels et dérivés), pyridoxine (sels et dérivés), cobalamine et dérivés, menadione et dérivés, acide folique et dérivés, acide ascorbique (sels et dérivés), acide nicotinique, nicotinamide, etc...
- acides aminés et protéines
- oligo-éléments (sélénium et ses sels, cuivre et ses sels, etc...)
- antibiotiques, tels que les macrolides, les tétracyclines, les pénicillines, les céphalosporines, les aminoglycosides,
- anti-infectieux de synthèse,
- antiparasitaires,
- facteurs de croissance,
- autres antibactériens,
- antifongiques,
- antiseptiques, etc...

Selon un autre mode de réalisation avantageux de l'invention, la composition d'enrobage comprend, en outre, au moins un autre constituant, choisi parmi les plastifiants et les agents anti-adhésion.

Selon une disposition avantageuse de ce mode de réalisation, les plastifiants sont choisis parmi la glycérine et ses esters, les polyéthylènes glycols de haut poids moléculaire, l'huile de ricin, les esters des acides citrique, phtalique, adipique, sébacique.

Selon une autre disposition avantageuse de ce mode de réalisation, les agents anti-adhésion sont préférentiellement choisis parmi le talc, la silice colloïdale, le stéarate de magnésium.

Selon encore un autre mode de réalisation avantageux dudit principe actif enrobé (microparticules), il comprend au moins 50 % en poids de principe actif.

La présente invention a également pour objet des compositions granulées ou compactées, caractérisées en ce qu'elles comprennent au moins un principe actif stabilisé pulvérulent tel que défini ci-dessus.

Les principes actifs stabilisés conformes à l'invention et les compositions granulées ou compactées (y compris les mélanges de poudres) les contenant, trouvent application dans le domaine pharmaceutique, vétérinaire, diététique, alimentaire ou cosmétique.

La présente invention a également pour objet un procédé de stabilisation de principes actifs, caractérisé :
- en ce que l'on prépare une composition d'enrobage par mise en solution, suspension ou émulsion dans un véhicule ou un mélange de véhicules inertes vis-à-vis du principe actif :
   . d'au moins un agent filmogène, dans des proportions comprises entre 2 et 25 % en poids de la masse finale, sélectionné parmi les polyvinylpyrrolidones (povidone), les alcools polyvinyliques, le copolymère vinylpyrrolidone - acétate de vinyle, le copolymère vinylpyrrolidone - alcool polyvinylique, les dérivés cellulosiques tels que cellulose acétate, cellulose acétate phtalate, cellulose butyrate, éthylcellulose, méthylcellulose, les polymères et copolymères acryliques et méthacryliques et les cires végétales, (Carnauba, Candellila...), animales (abeille, huile de ricin hydrogénée...) ou synthétiques (polymères éthyléniques, polyol ether-ester tel que Carbowax®...), et
   . d'au moins un agent porogène, dans des proportions comprises entre 0 et 5 %, de préférence entre 0,5 et 5 %, en poids de la masse finale, sélectionné parmi le lactose microcristallin, les polyéthylènes glycols de bas poids moléculaire, le carbonate de calcium, le phosphate de calcium, le saccharose, le chlorure de sodium et le chlorure de potassium,
- en ce que l'on enrobe le principe actif sous forme pulvérulente, avec ladite composition d'enrobage en solution, en suspension ou en émulsion,
- et en ce que l'on obtient un principe actif pulvérulent stabilisé, enrobé dans ladite composition d'enrobage (microparticules), présentant une granulométrie comprise entre 50 et 1 000 µm, de préférence entre 200 et 500 µm.

Selon un mode de mise en oeuvre avantageux dudit procédé, l'enrobage est réalisé par pulvérisation en lit d'air fluidisé.

Selon un autre mode de mise en oeuvre avantageux dudit procédé, l'enrobage est réalisé par nébulisation.

L'invention inclut également, comme technique d'enrobage, toute autre technique permettant d'obtenir le même résultat (film homogène, propriétés physiques conservées).

Préalablement audit enrobage, la composition d'enrobage est avantageusement en solution, en suspension ou en émulsion dans un véhicule ou mélange de véhicules inertes vis-à-vis du principe actif à enrober, comme précisé ci-dessus. Le choix du véhicule, utilisé seul ou en association, est fonction des propriétés de l'agent filmogène et, éventuellement de l'agent porogène et de l'agent séquestrant utilisé.

Les véhicules sont préférentiellement choisis parmi l'eau, les hydrocarbures halogénés tels que le chlorure de méthylène, le chloroforme, le dichloroéthane..., les alcools tels que le méthanol, l'éthanol, le propanol, l'isopropanol et l'acétone.

Conformément à l'invention, préalablement à l'enrobage, ladite composition d'enrobage est associée à au moins un autre constituant, choisi parmi les plastifiants et les agents anti-adhésion, tels que définis ci-dessus.

Outre les dispositions qui précédent, l'invention comprend encore d'autres dispositions qui ressortiront de la description qui va suivre, se référant aux exemples de compositions et de mise en oeuvre du procédé, objets de la présente invention.

Il doit être bien entendu, toutefois, que ces exemples sont donnés uniquement à titre d'illustration de l'objet de l'invention dont ils ne constituent en aucune manière une limitation.

### EXEMPLE 1 : Préparation de microparticules de furaltadone (principe actif) stabilisée par une composition d'enrobage comprenant comme agent filmogène, de la povidone et comme agent porogène, du chlorure de sodium (enrobage par traitement en lit d'air fluidisé).

3 kg de furaltadone sont mis en suspension dans un granulateur GLATT WSG5, et traités par une solution de Povidone, 220 g, et de chlorure de sodium, 30 g, à 6 % m/v en milieu hydroalcoolique (eau 80/éthanol 20).

Le traitement s'effectue dans les conditions suivantes :
- débit de pulvérisation 40 ml/min,
- pression d'atomisation 1,2 à 1,4 bar,
- température dans la chambre 50 à 55°C,
- température du produit 30 à 35°C,
- débit d'air 1,5 à 2 m³/min.

A l'issue de la pulvérisation, la circulation d'air est maintenue dans les mêmes conditions de température et de pression, pendant 45 à 60 minutes pour assurer un bon séchage. Le produit obtenu présente une granulométrie comprise entre 0,2 et 0,5 mm.

### EXEMPLE 2 : Préparation de microparticules d'acide ascorbique stabilisé par une composition d'enrobage comprenant comme agent séquestrant, de la dextrine, comme agent filmogène, du polyméthacrylate, et comme agent porogène, du saccharose (enrobage par nébulisation).

Un mélange homogène de 250 g d'acide ascorbique et 50 g de dextrine est mis en suspension dans 2 litres de chlorure de méthylène contenant 40 g de polyméthacrylate et 10 g de saccharose. L'atomisation est conduite sur un atomiseur MINOR MOBILE (NIRO-ATOMIZER) équipé d'une buse à jet ascendant, dans les conditions suivantes :
- débit d'alimentation 30 ml/min,
- pression de 3 à 3,2 bars,
- température de la chambre 28 à 32°C.

Le produit obtenu a une granulométrie comprise entre 0,08 et 0,2 mm (microparticules 2A).

Des microparticules d'acide ascorbique stabilisé par une composition d'enrobage sans agent porogène peuvent également être obtenues dans les mêmes conditions (microparticules 2B).

### EXEMPLE 3 : Préparation de microparticules d'amoxicilline stabilisée avec une composition d'enrobage contenant comme agent filmogène, de la povidone, comme agent porogène, du lactose, et comme autres constituants, un plastifiant et un agent anti-adhésion (enrobage par traitement en lit d'air fluidisé).

3 kg d'amoxicilline trihydrate sont mis en suspension dans un granulateur GLATT WSG5, et traités par une solution de povidone, 180 g à 6 % m/v en milieu hydroalcoolique (eau 80/éthanol 20), en présence de 35 g de lactose, 30 g de dibutylphtalate et 5 g de silice colloïdale.

Le traitement s'effectue dans les conditions suivantes :
- débit de pulvérisation 40 ml/min,
- pression d'atomisation 1,2 à 1,4 bar,
- température dans la chambre 50 à 55°C,
- température du produit 30 à 35°C,
- débit d'air 1,5 à 2 m³/min.

A l'issue de la pulvérisation, la circulation d'air est maintenue dans les mêmes conditions de température et de pression, pendant 45 à 60 minutes pour assurer un bon séchage. Le produit obtenu présente une granulométrie comprise entre 0,2 et 0,5 mm (microparticules 3A).

Des microparticules d'amoxicilline stabilisée avec une composition d'enrobage sans agent porogène peuvent également être obtenues dans les mêmes conditions que ci-dessus (microparticules 3B).

### EXEMPLE 4 : Préparation d'une composition granulée par extrusion, comprenant des microparticules selon l'exemple 3.

Les microparticules selon l'exemple 3, sont transformées classiquement en granulés par extrusion, en présence de farines végétales telles que la farine de blé ou de soja. Cette fabrication fait intervenir des opérations de mélanges à sec, puis en présence d'eau, de compression en présence de vapeur d'eau et de séchage.

Les granulés contenant 400 ppm d'amoxicilline, conservés dans des conditions normales de température (22°C environ) et d'humidité (70 % environ) dans l'emballage de vente (sac de papier), sont analysés périodiquement ; parallèlement des granulés réalisés de la même manière, avec de l'amoxicilline non stabilisée, conservés dans les mêmes conditions, sont analysés. Les résultats sont rassemblés dans le Tableau I ci-après :

**Tableau I**

| Durée de conservation | Initial | 20 jours | 43 jours | 100 jours | 170 jours |
|---|---|---|---|---|---|
| teneur en amoxicilline Granulés selon l'invention | 411 | 398 | 404 | 399 | 396 |
| teneur en amoxicilline Granulés de référence | 323 | 285 | 226 | 167 | 132 |

Cette expérimentation met en évidence la stabilité du principe actif (amoxicilline) dans un produit manufacturé (granulés) mettant en oeuvre de l'amoxicilline stabilisée (microparticules) conforme à l'invention.

### EXEMPLE 5 : Test de disponibilité in vitro.

Un test de disponibilité *in vitro* de l'amoxicilline, à partir des granulés préparés selon l'exemple 4 (microparticules 3A et 3B) a été réalisé comparativement à des granulés d'amoxicilline (granulés référence 1) préparés avec de l'amoxicilline non stabilisée (fabrication classique) :

**Tableau II**

| Durée de dissolution | 30 s | 1 min | 5 min | 10 min |
|---|---|---|---|---|
| % d'amoxicilline solubilisé Granulés 3A de l'exemple 4 | 51 | 63 | 96 | 100 |
| % d'amoxicilline solubilisé Granulés 3B de l'exemple 4 | 40 | 61 | 96 | 100 |
| % d'amoxicilline solubilisé Granulés 3A de référence 1 | 50 | 59 | 95 | 100 |

Cette expérimentation montre que la disponibilité du principe actif (amoxicilline) dans un produit manufacturé (granulés) réalisé selon l'invention est en tout point équivalente à celle d'un granulé contenant le même principe actif non enrobé ; ceci montre bien que les caractéristiques physiques du principe actif ne sont pas modifiées par l'enrobage de protection ; en outre, notamment pour des durées très courtes, la présence d'agent porogène permet une solubilisation du principe actif, à la même vitesse que celle du principe actif non enrobé.

### EXEMPLE 6 : Autre test de disponibilité in vitro.

Un test de disponibilité *in vitro* de l'acide ascorbique, dans un mélange de poudres (A), préparé à partir des microparticules 2A selon l'exemple 2 a été réalisé, comparativement à un mélange de poudres (B) préparé à partir de microparticules 2B selon l'exemple 2 et à un mélange de poudres (C) préparé avec de l'acide ascorbique non stabilisé (non enrobé). Les résultats sont rassemblés dans le Tableau III.

**Tableau III**

| Durée de dissolution | 30 s | 1 min | 5 min |
|---|---|---|---|
| % d'acide ascorbique solubilisé Mélange de poudres (A) | 85 | 98 | 100 |
| % d'acide ascorbique solubilisé Mélange de poudres (B) | 65 | 91 | 100 |
| % d'acide ascorbique solubilisé Mélange de poudres (C) | 98 | 100 | |

Cette expérimentation montre que la disponibilité du principe actif (acide ascorbique) dans un mélange de poudres préparé avec des microparticules selon l'invention est en tout point équivalente à celle d'un mélange de poudres contenant le même principe actif non enrobé.

## Revendications

1. Principes actifs pulvérulents stabilisés par enrobage avec une composition d'enrobage du type comprenant au moins un agent filmogène, caractérisés :
(i) en ce que ladite composition d'enrobage comprend :
- au moins un agent filmogène, dans des proportions comprises entre 2 et 25 % en poids de la masse finale, sélectionné parmi les polyvinylpyrrolidones (povidone), les alcools polyvinyliques, le copolymère vinylpyrrolidone - acétate de vinyle, le copolymère vinylpyrrolidone - alcool polyvinylique, les dérivés cellulosiques tels que cellulose acétate, cellulose acétate phtalate, cellulose butyrate, éthylcellulose, méthylcellulose, les polymères et copolymères acryliques et méthacryliques et les cires végétales, animales ou synthétiques, et
- au moins un agent porogène, dans des proportions comprises entre 0 et 5 % en poids de la masse finale, sélectionné parmi le lactose microcristallin, les polyéthylènes glycols de bas poids moléculaire, le carbonate de calcium, le phosphate de calcium, le saccharose, le chlorure de sodium et le chlorure de potassium,
(ii) et en ce que le produit actif pulvérulent stabilisé sous la forme de microparticules, présente une granulométrie comprise entre 50 et 1 000 µm, de préférence entre 200 et 500 µm.

2. Principes actifs stabilisés selon la revendication 1, caractérisés en ce que l'agent porogène est dans des proportions comprises entre 0,5 et 5 % en poids de la masse finale.

3. Principes actifs stabilisés selon la revendication 1 ou la revendication 2, caractérisés en ce que ledit principe actif sous forme pulvérulente est associé à un agent séquestrant choisi parmi les carbohydrates ou polysaccharides tels que cellulose, dextrines, cyclodextrines, amidon, dextranes...

4. Principes actifs stabilisés selon l'une quelconque des revendications 1 à 3, caractérisés en ce que ladite composition d'enrobage comprend, en outre, au moins l'un des autres constituants suivants :
- des plastifiants choisis parmi la glycérine et ses esters, les polyéthylènes glycols de haut poids moléculaire, l'huile de ricin, les esters des acides citrique, phtalique, adipique, sébacique, et
- des agents anti-adhésion choisis parmi le talc, la silice colloïdale, le stéarate de magnésium.

5. Principes actifs stabilisés selon l'une quelconque des revendications 1 à 4, caractérisés en ce qu'ils comprennent au moins 50 % en poids de principe actif.

6. Compositions granulées ou compactées, caractérisées en ce qu'elles comprennent au moins un principe actif stabilisé pulvérulent selon l'une quelconque des revendications 1 à 5.

7. Procédé de stabilisation de principes actifs, caractérisé :
- en ce que l'on prépare une composition d'enrobage par mise en solution, suspension ou émulsion dans un véhicule ou un mélange de véhicules inertes vis-à-vis du principe actif :
. d'au moins un agent filmogène, dans des proportions comprises entre 2 et 25 % en poids de la masse finale, sélectionné parmi les polyvinylpyrrolidones (povidone), les alcools polyvinyliques, le copolymère vinylpyrrolidone - acétate de vinyle, le copolymère vinylpyrrolidone - alcool polyvinylique, les dérivés cellulosiques tels que cellulose acétate, cellulose acétate phtalate, cellulose butyrate, éthylcellulose, méthylcellulose, les polymères et copolymères acryliques et méthacryliques et les cires végétales, animales ou synthétiques, et
. d'au moins un agent porogène, dans des proportions comprises entre 0 et 5 %, de préférence entre 0,5 et 5 %, en poids de la masse finale, sélectionné parmi le lactose microcristallin, les polyéthylènes glycols de bas poids moléculaire, le carbonate de calcium, le phosphate de calcium, le saccharose, le chlorure de sodium et le chlorure de potassium,
- en ce que l'on enrobe le principe actif sous forme pulvérulente, avec ladite composition d'enrobage en solution, en suspension ou en émulsion,
- et en ce que l'on obtient un principe actif pulvérulent stabilisé, enrobé dans ladite composition d'enrobage (microparticules), présentant une granulométrie comprise entre 50 et 1 000 µm, de préférence entre 200 et 500 µm.

8. Procédé selon la revendication 7, caractérisé en ce que l'étape d'enrobage est réalisée en lit d'air fluidisé.

9. Procédé selon la revendication 7, caractérisé en ce que l'étape d'enrobage est réalisée par nébulisation.

10. Procédé selon l'une quelconque des revendications 7 à 9, caractérisé en ce que la composition d'enrobage comprend, en outre, au moins un autre constituant, choisi parmi les plastifiants et les agents anti-adhésion tels que définis ci-dessus, à la revendication 4.

11. Application des principes actifs selon l'une quelconque des revendications 1 à 5 et de la composition selon la revendication 6, à la préparation d'un médicament.

12. Application des principes actifs selon l'une quelconque des revendications 1 à 5 et de la composition selon la revendication 6, à la cosmétologie.

13. Application des principes actifs selon l'une quelconque des revendications 1 à 5 et de la composition selon la revendication 6, à la préparation d'un produit alimentaire ou diététique.

14. Composition d'enrobage de principes actifs, caractérisée en ce qu'elle comprend :
- au moins un agent filmogène, sélectionné parmi les polyvinylpyrrolidones, les alcools polyvinyliques, le copolymère vinylpyrrolidone - acétate de vinyle, le copolymère vinylpyrrolidone - alcool polyvinylique, les dérivés cellulosiques tels que cellulose acétate, cellulose acétate phtalate, cellulose butyrate, éthylcellulose, méthylcellulose, les polymères et copolymères acryliques et méthacryliques et les cires végétales, animales ou synthétiques, et
. au moins un agent porogène, sélectionné parmi le lactose microcristallin, les polyéthylènes glycols de bas poids moléculaire, le carbonate de calcium, le phosphate de calcium, le saccharose, le chlorure de sodium et le chlorure de potassium.

15. Composition d'enrobage selon la revendication 14, caractérisée en ce qu'elle comprend en outre, au moins l'un des autres constituants suivants :
- des plastifiants choisis parmi la glycérine et ses esters, le polyéthylène glycol, l'huile de ricin, les esters des acides citrique, phtalique, adipique, sébacique, et
- des agents anti-adhésion choisis parmi le talc, la silice colloïdale, le stéarate de magnésium.

## Claims

1. Pulverulent active agents stabilized by coating with a coating composition of the type comprising at least one film-forming agent, characterized:
(i) in that the said coating composition comprises:
- at least one film-forming agent, in proportions of between 2 and 25% by weight of the final mass, selected from polyvinylpyrrolidones (povidone), polyvinyl alcohols, vinylpyrrolidone/vinyl acetate copolymer, vinylpyrrolidone/polyvinyl alcohol copolymer, cellulose derivatives such as cellulose acetate, cellulose acetate phthalate, cellulose butyrate, ethylcellulose and methylcellulose, acrylic and methacrylic polymers and copolymers and vegetable, animal or synthetic waxes, and
- at least one pore-forming agent, in proportions of between 0 and 5% by weight of the final mass, selected from microcrystalline lactose, low molecular weight polyethylene glycols, calcium carbonate, calcium phosphate, sucrose, sodium chloride and potassium chloride,
(ii) and in that the stabilized pulverulent active product in the form of microparticles possesses a particle size of between 50 and 1000 µm, and preferably between 200 and 500 µm.

2. Stabilized active agents according to Claim 1, characterized in that the pore-forming agent is in proportions of between 0.5 and 5% by weight of the final mass.

3. Stabilized active agents according to Claim 1 or Claim 2, characterized in that the said active agent in pulverulent form is combined with a sequestering agent chosen from carbohydrates or polysaccharides such as cellulose, dextrins, cyclodextrins, starch, dextrans, etc.

4. Stabilized active agents according to Claim 1, Claim 2 or Claim 3, characterized in that the said coating composition comprises, in addition, at least one of the following other constituents:
- plasticizers chosen from glycerol and its esters, high molecular weight polyethylene glycols, castor oil and citric, phthalic, adipic and sebacic acid esters, and
- antiadhesive agents chosen from talc, colloidal silica and magnesium stearate.

5. Stabilized active agents according to any one of Claims 1 to 4, characterized in that they comprise at least 50% of active agent.

6. Granulated or compacted compositions, characterized in that they comprise at least one pulverulent stabilized active agent according to any one of Claims 1 to 5.

7. Process for the stabilization of active agents, characterized:
- in that a coating composition is prepared by dissolution, suspension, or emulsification, in a vehicle or mixture of vehicles which is/are inert with respect to the active agent, of:
. at least one film-forming agent, in proportions of between 2 and 25% by weight of the final mass, selected from polyvinylpyrrolidones (povidone), polyvinyl alcohols, vinylpyrrolidone/vinyl acetate copolymer, vinylpyrrolidone/polyvinyl alcohol copolymer, cellulose derivatives such as cellulose acetate, cellulose acetate phthalate, cellulose butyrate, ethylcellulose and methylcellulose, acrylic and methacrylic polymers and copolymers and vegetable, animal or synthetic waxes, and eventually,
. at least one pore-forming agent, in proportions of between 0 and 5%, preferably of between 0.5 and 5% by weight of the final mass, selected from microcrystalline lactose, low molecular weight polyethylene glycols, calcium carbonate, calcium phosphate, sucrose, sodium chloride and potassium chloride,
- in that the active agent in pulverulent form is coated with the said coating composition in solution, suspension or emulsion,
- and in that a stabilized pulverulent active agent, coated in the said coating composition (microparticles), possessing a particle size of between 50 and 1,000 µm and preferably between 200 and 500 µm, is obtained.

8. Process according to Claim 7, characterized in that the coating step is carried out in an air-fluidized bed.

9. Process according to Claim 7, characterized in that the coating step is carried out by nebulization.

10. Process according to any one of Claims 7 to 9, characterized in that the coating composition comprises, in addition, at least one other constituent chosen from plasticizers and antiadhesive agents as are defined above in Claim 4.

11. Application of the active agents according to any one of Claims 1 to 5 and of the composition according to Claim 6 to the preparation of a medicinal product.

12. Application of the active agents according to any one of Claims 1 to 5 and of the composition according to Claim 6 to cosmetology.

13. Application of the active agents according to any one of Claims 1 to 5 and of the composition according to Claim 6 to the preparation of a nutritional or dietary product.

14. Coating composition for active agents, characterized in that it comprises:
- at least one film-forming agent selected from polyvinylpyrrolidones, polyvinyl alcohols, vinylpyrrolidone/vinyl acetate copolymer, vinylpyrrolidone/polyvinyl alcohol copolymer, cellulose derivatives such as cellulose acetate, cellulose acetate phthalate, cellulose butyrate, ethylcellulose and methylcellulose, acrylic and methacrylic polymers and copolymers and vegetable, animal or synthetic waxes, and
- at least one pore-forming agent selected from microcrystalline lactose, low molecular weight polyethylene glycols, calcium carbonate, calcium phosphate, sucrose, sodium chloride and potassium chloride.

15. Coating composition according to Claim 14, characterized in that it comprises, in addition, at least one of the following other constituents:
- plasticizers chosen from glycerol and its esters, polyethylene glycol, castor oil and citric, phthalic, adipic and sebacic acid esters, and
- antiadhesive agents chosen from talc, colloidal silica and magnesium stearate.

## Patentansprüche

1. Pulverförmige Wirkstoffe, stabilisiert durch Ummantelung mit einer derartigen Mantelzusammensetzung, welche zumindest ein filmbildendes Mittel enthält, dadurch gekennzeichnet, daß:
(i) die Mantelzusammensetzung
- zumindest ein filmbildendes Mittel in Anteilen zwischen 2 und 25 Gew.-% der Endmasse, ausgewählt aus den Polyvinylpyrrolidonen (Povidon), den Polyvinylalkoholen, dem Copolymeren Vinylpyrrolidon-Vinylacetat, dem Copolymeren Vinylpyrrolidon-Polyvinylalkohol, den Zellulosederivaten, wie Zelluloseacetat, Zelluloseacetatphtalat, Zellulosebutyrat, Ethylzellulose, Methylzellulose, den Akryl- und Methakryl-Polymeren und -Copolymeren und den pflanzlichen, tierischen oder synthetischen Wachsen, und
- zumindest ein porenbildendes Mittel in Anteilen zwischen 0 und 5 Gew.-% der Endmasse, ausgewählt aus der mikrokristallinen Laktose, den Polyethylenglycolen mit niedrigem Molekulargewicht, dem Kalziumcarbonat, dem Kalziumphosphat, der Saccharose, dem Natriumchlorid und dem Kaliumchlorid, enthält,
(ii) und daß das wirksame pulverförmige Produkt, stabilisiert in Form von Mikropartikeln, eine Granulatgröße zwischen 50 und 1000 µm, vorzugsweise zwischen 200 und 500 µm, aufweist.

2. Wirkstoffe, stabilisiert nach Anspruch 1, dadurch gekennzeichnet, daß das porenbildende Mittel in Anteilen zwischen 0,5 und 5 Gew.-% der Endmasse vorliegt.

3. Wirkstoffe, stabilisiert nach Anspruch 1 oder Anspruch 2, dadurch gekennzeichnet, daß der Wirkstoff in Pulverform mit einem Sequestrierungsmittel, ausgewählt aus den Kohlenhydraten oder Polysacchariden, wie Zellulose, Dextrine, Cyclodextrine, Amidon, Dextrane ... verbunden ist.

4. Wirkstoffe, stabilisiert nach einem der Ansprüche 1 bis 3, dadurch gekennzeichnet, daß die Mantelzusammensetzung unter anderem zumindest einen der folgenden anderen Bestandteilen enthält:
- Weichmacher, ausgewählt aus dem Glycerin und seinen Estern, den Polyethylenglycolen mit hohem Molekulargewicht, dem Rizinusöl, den Estern der Zitronen-, Phtal- Adipin-, Sebacinsäuren, und
- Antihaftmittel, ausgewählt aus Talk, kolloidaler Kieselerde, Magnesiumstearat.

5. Wirkstoffe, stabilisiert nach einem der Ansprüche 1 bis 4, dadurch gekennzeichnet, daß sie zumindest 50 Gew.-% Wirkstoff enthalten.

6. Granulierte oder gepreßte Zusammensetzungen, dadurch gekennzeichnet, daß sie zumindest einen stabilisierten, pulverförmigen Wirkstoff nach einem der Ansprüche 1 bis 5 enthalten.

7. Verfahren zur Wirkstoffstabilisierung, dadurch gekennzeichnet, daß man
- eine Mantelzusammensetzung herstellt, indem man
. zumindest ein filmbildendes Mittel in Anteilen zwischen 2 und 25 Gew.-% der Endmasse, ausgewählt aus den Polyvinylpyrrolidonen (Povidon), den Polyvinylalkoholen, dem Copolymeren Vinylpyrrolidon-Vinylacetat, dem Copolymeren Vinylpyrrolidon-Polyvinylalkohol, den Zellulosederivaten, wie Zelluloseacetat, Zelluloseacetatphtalat, Zellulosebutyrat, Ethylzellulose, Methylzellulose, den Akryl- und Methakryl-Polymeren und -Copolymeren und den pflanzlichen, tierischen oder synthetischen Wachsen, und
. zumindest ein porenbildendes Mittel in Anteilen zwischen 0 und 5 Gew.-% der Endmasse, ausgewählt aus der mikrokristallinen Laktose, den Polyethylenglycolen mit niedrigem Molekulargewicht, dem Kalziumcarbonat, dem Kalziumphosphat, der Saccharose, dem Natriumchlorid und dem Kaliumchlorid, in einem gegenüber dem Wirkstoff inerten Träger oder Trägergemisch in Lösung, Suspension oder Emulsion bringt,
- den Wirkstoff in Pulverform mit der Mantelzusammensetzung in Losung, Suspension oder Emulsion ummantelt,
- und einen pulverförmigen, stabilisierten Wirkstoff, ummantelt mit der Mantelzusammensetzung (Mikroteilchen), erhält, der eine Granulatgröße zwischen 50 und 1000 µm, vorzugsweise zwischen 200 und 500 µm aufweist.

8. Verfahren nach Anspruch 7, dadurch gekennzeichnet, daß die Ummantelungsstufe in einem Luftwirbelbett durchgeführt wird.

9. Verfahren nach Anspruch 7, dadurch gekennzeichnet, daß die Ummantelungsstufe durch Bestäuben durchgeführt wird.

10. Verfahren nach einem der Ansprüche 7 bis 9, dadurch gekennzeichnet, daß die Mantelzusammensetzung unter anderem zumindest einen anderen Bestandteil, ausgewählt aus den Weichmachern und den Antihaftmitteln, wie oben bei Anspruch 4 definiert, enthält.

11. Anwendung der Wirkstoffe nach einem der Ansprüche 1 bis 5 und der Zusammensetzung nach Anspruch 6 zur Herstellung eines Medikaments.

12. Anwendung der Wirkstoffe nach einem der Ansprüche 1 bis 5 und der Zusammensetzung nach Anspruch 6 in der Kosmetologie.

13. Anwendung der Wirkstoffe nach einem der Ansprüche 1 bis 5 und der Zusammensetzung nach Anspruch 6 zur Herstellung eines zur Nahrung dienenden oder dieätetischen Produkts.

14. Zusammensetzung zur Ummantelung von Wirkstoffen, dadurch gekennzeichnet, daß sie:
. zumindest ein filmbildendes Mittel, ausgewählt aus den Polyvinylpyrrolidonen, den Polyvinylalkoholen, dem Copolymeren Vinylpyrrolidon-Vinylacetat, dem Copolymeren Vinylpyrrolidon-Polyvinylalkohol, den Zellulosederivaten, wie Zelluloseacetat, Zelluloseacetatphtalat, Zellulosebutyrat, Ethylzellulose, Methylzellulose, den Akryl- und Methakryl-Polymeren und -Copolymeren und den pflanzlichen, tierischen oder synthetischen Wachsen, und
. zumindest ein porenbildendes Mittel, ausgewählt aus der mikrokristallinen Laktose, den Polyethylenglycolen mit niedrigem Molekulargewicht, dem Kalziumcarbonat, dem Kalziumphosphat, der Saccharose, dem Natriumchlorid und dem Kaliumchlorid,
enthält.

15. Mantelzusammensetzung nach Anspruch 14, dadurch gekennzeichnet, daß sie unter anderem zumindest einen der anderen folgenden Bestandteile enthält:
- Weichmacher, ausgewählt aus dem Glycerin und seinen Estern, dem Polyethylenglycol, dem Rizinusöl, den Estern der Zitronen-, Phtal- Adipin-, Sebacinsäuren, und
- Antihaftmittel, ausgewählt aus Talk, kolloidaler Kieselerde, Magnesiumstearat.
